# EUROPEAN PATENT SPECIFICATION

(11) **EP 3 989 883 B1**
(45) Date of publication and mention of the grant of the patent: **29.11.2023**
(21) Application number: 20736473.8
(22) Date of filing: 23.06.2020
(51) Int. Cl.: A61F 2/30, A61L 27/18, A61L 27/56

(54) **PLUG-SHAPED IMPLANT FOR THE REPLACEMENT AND REGENERATION OF BIOLOGICAL TISSUE AND METHOD FOR PREPARING THE IMPLANT**
STOPFENFÖRMIGES IMPLANTAT ZUM ERSETZEN UND REGENERIEREN VON BIOLOGISCHEM GEWEBE UND VERFAHREN ZUR HERSTELLUNG DES IMPLANTATS
IMPLANT EN FORME DE BOUCHON POUR LE REMPLACEMENT ET LA RÉGÉNÉRATION D'UN TISSU BIOLOGIQUE ET PROCÉDÉ DE PRÉPARATION DE L'IMPLANT

(30) Priority: 27.06.2019 NL 2023398
(43) Date of publication of application: 04.05.2022
(73) Proprietor: Jointsphere B.V., 5232 AC 's-Hertogenbosch (NL)
(72) Inventor: HERMSEN, Egidius Gerardus Maria, 5611 CZ Eindhoven (NL); VAN BUUL, Everardus Johannes Hubertus, 5212 AR 's-Hertogenbosch (NL); MELSOM, Giles William, 6721 NP Bennekom (NL); FRANSEN, Petrus Mattheus Egidius Adrianus, 5241 MB Rosmalen (NL)
(74) Representative: Arnold & Siedsma
(86) International application number: PCT/NL2020/050412
(87) International publication number: WO 2020/263087

(56) References cited:
- US-A1- 2005 013 793
- US-A1- 2008 262 618
- US-A1- 2009 164 014
- US-A1- 2012 209 396

## Description

### TECHNICAL FIELD OF THE INVENTION

The invention relates to an implant for the replacement and regeneration of biological tissue in the shape of a plug. The invention in particular relates to an implant for the replacement and regeneration of an osteochondral structure in the shape of a plug. The invention further relates to a method for the preparation of the implant.

### BACKGROUND OF THE INVENTION

An osteochondral structure refers to a structure comprising cartilage and bone. Typical osteochondral structures can be found in the thighbone (femur), shinbone (tibia), and kneecap (patella). Such structures fit tightly together and move smoothly because the bone surface is covered with a relatively thick layer of articular (hyaline) cartilage. An (osteo)chondral defect is any type of damage to articular cartilage and optionally to underlying (subchondral) bone. Usually, (osteo)chondral defects appear on specific weight-bearing spots at the ends of the thighbone and shinbone and the back of the kneecap for instance. They may range from roughened cartilage, small bone and cartilage fragments that hinder movement, to complete cartilage loss.

Trauma of joint surfaces is common in young active people practicing sports, or as a sequel to accidents. Lesions may comprise the cartilage layer only, but often the underlying subchondral bone too. Articular cartilage has a very low tendency for healing and the repair tissue is qualitatively inferior to the original tissue. This invariably leads to the formation of osteoarthritis (OA) over the years, which is a major cause of disability and loss of quality of life in elderly people. The standard treatment for this condition is ultimately joint replacement by artificial joints. Whilst clinically effective, the non-biological implants do not last longer than 10-20 years and revision surgery is much less effective and very costly. For this reason, much research is dedicated to developing biological regenerative therapies that would be life-long lasting. However, despite promising in vitro results, until now not a single solution has proven to be more effective than the current standard of care over a longer period in real life conditions.

Because the cartilage layer lacks nerve fibers, patients are often not aware of the severity of the damage. During the final stage, an affected joint consists of bone rubbing against bone, which leads to severe pain and limited mobility. By the time patients seek medical treatment, surgical intervention may be required to alleviate pain and repair the cartilage damage. Implants have been developed for the joint in order to avoid or postpone such surgical interventions. These may be implanted in a bone structure at an early stage of cartilage damage, and may thus be provided for preventive treatment, in order to avoid unnoticed degeneration of the joint.

A number of treatments is available to treat articular cartilage damage in joints, such as the knee, starting with the most conservative, non-invasive options and ending with total joint replacement if the damage has spread throughout the joint. Currently available treatments include anti-inflammatory medications in the early stages. Although these may relieve pain, they have limited effect on arthritis symptoms and further do not repair joint tissue. Cartilage repair methods, such as arthroscopic debridement, attempt to at least delay tissue degeneration. These methods however are only partly effective at repairing soft tissue, and do not restore joint spacing or improve joint stability. Joint replacement (arthroplasty) is considered as a final solution, when all other options to relieve pain and restore mobility have failed or are no longer effective. While joint arthroplasty may be effective, the procedure is extremely invasive, technically challenging and may compromise future treatment options. Cartilage regeneration has also been attempted, more in particular by tissue-engineering technology. The use of cells, genes and growth factors combined with scaffolds plays a fundamental role in the regeneration of functional and viable articular cartilage. All of these approaches are based on stimulating the body's normal healing or repair processes at a cellular level. Many of these compounds are delivered on a variety of carriers or matrices including woven polylactic acid based polymers or collagen fibers. Despite various attempts to regenerate cartilage, a reliable and proven treatment does not currently exist for repairing defects to the articular cartilage.

Another standard of care consists of Microfracture (MFx) for smaller lesions (≤ 2 cm²) and Autologous Chondrocyte Implantation (ACI) for bigger lesions (> 2 cm2). The cartilaginous tissue regenerated with these techniques however is not able to withstand the biomechanical challenges in the joint and starts to degenerate within 18 months already. Substantial delay in joint replacement by artificial joints, let alone preventing it, therefore is not possible.

US2009/164014A1 discloses a biodegradable implant in the shape of a plug.

### SUMMARY OF THE INVENTION

It is an object of the present invention to provide an implant for the replacement and regeneration of biological tissue in the shape of a plug having improved load distribution as well as cartilage regenerating properties. Another aim is to provide such a plug-shaped implant for the replacement and regeneration of an osteochondral structure. Yet another aim is to provide a method for the preparation of the implant. The invention further aims to provide an implant which is able to repair articular cartilage lesions in a durable fashion, and which at least postpones and, preferably, prevents joint replacement by artificial joints.

The above and other aims are provided by a plug-shaped implant in accordance with claim 1. The plug-shaped non-biodegradable implant in particular comprises a base section configured for anchoring in bone tissue, a middle section configured for replacing cartilage tissue of an intermediate and deep zone of the cartilage layer and having a thickness of at least 0.2 mm, and a top section configured for growing cartilage tissue onto and into, thus regenerating a superficial zone of the cartilage layer, wherein the middle and top section comprise the same non-biodegradable thermoplastic elastomeric material, which is porous in the top section, and non-porous in the middle section, wherein the thermoplastic elastomeric material comprises a linear block copolymer comprising urethane and urea groups, and is substantially free of an added peptide compound having cartilage regenerative properties, and wherein the base section material comprises one of a biocompatible metal, ceramic, mineral, such as phosphate mineral, and a non-biodegradable polymer, optionally a hydrogel polymer, and combinations thereof.

Preferably, the thermoplastic elastomeric material is substantially free of any added compound having cartilage regenerative properties.

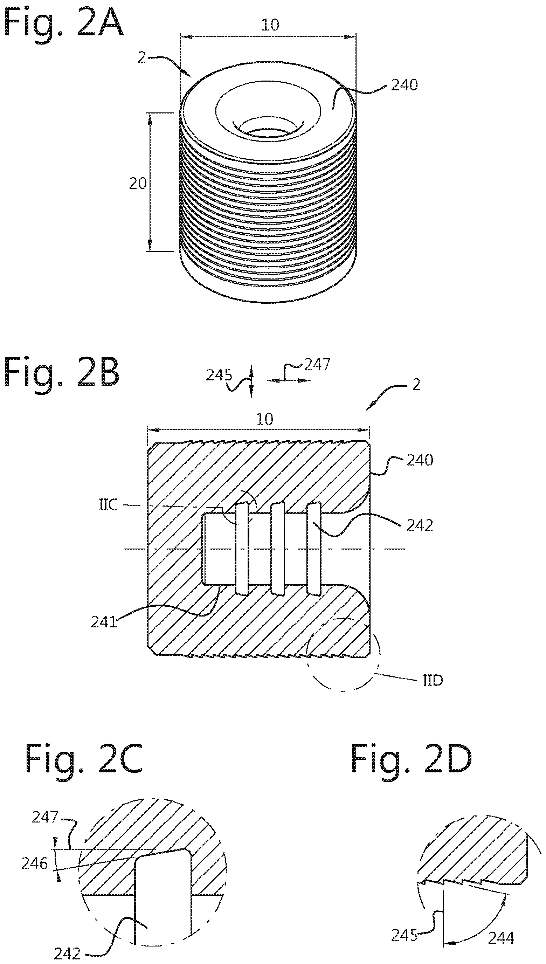

In cartilage, a relatively thin superficial (tangential) zone protects deeper layers from shear stresses and makes up approximately 10% to 20% of articular cartilage thickness. The collagen fibers of this zone (primarily, type II and IX collagen) are packed tightly and aligned parallel to the articular surface (Figure 2). The superficial layer contains a relatively high number of flattened chondrocytes, and the integrity of this layer is imperative in the protection and maintenance of deeper layers. This zone is in contact with synovial fluid and is responsible for most of the tensile properties of cartilage, which enables it to resist the shear, tensile, and compressive forces imposed by articulation.

Immediately deep or below to the superficial zone is the middle (intermediate or transitional) zone, which provides an anatomic and functional bridge between the superficial and deep zones. The middle zone represents 40% to 60% of the total cartilage volume, and it contains proteoglycans and thicker collagen fibrils. In this layer, the collagen is organized obliquely, and the chondrocytes are spherical and at low density. Functionally, the middle zone is the first line of resistance to compressive forces.

The deep zone of cartilage is responsible for providing the greatest resistance to compressive forces, given that collagen fibrils are arranged perpendicular to the articular surface. The deep zone contains the largest diameter collagen fibrils in a radial disposition, the highest proteoglycan content, and the lowest water concentration. The chondrocytes are typically arranged in columnar orientation, parallel to the collagen fibers and perpendicular to the joint line. The deep zone represents approximately 30% of articular cartilage volume.

The base section material may be formed of any suitable material which provides an appropriate level of mechanical support to the surrounding bone and preferably allows osteogenesis. Suitable materials, including the thermoplastic elastomeric material of the middle and top section of the implant, are biocompatible, by which is meant that these materials are capable of coexistence with living tissues or organisms without causing harm to them. Further, the implant in accordance with the invention is substantially non-biodegradable and combines cartilage replacement with cartilage regeneration. With a non-biodegradable material in the context of the present invention is meant a material that is not broken down into less complex compounds or compounds having fewer carbon atoms by the environment of the implanted implant. The weight-average molecular weight of a substantially non-biodegradable material is reduced by at most 20%, relative to the original weight-average molecular weight after one year of implantation, more preferably at most 10%, still more preferably at most 5%, and more preferably still at most 1%.

Suitable metals as base section material include but are not limited to titanium, zirconium, chromium, aluminum, stainless steel, hafnium, tantalum or molybdenum, and their alloys, or any combination thereof. Optionally, a surface layer of the metal may be oxidized, nitrided, carburized or boronized to form a coated metal base section.

Suitable ceramics and minerals as base section material include but are not limited to oxides, nitrides, carbides or borides, or any combination thereof. Suitable examples include bioactive glass, calcium phosphates, such as beta-tricalcium phosphate (TCP), biphasic calcium phosphate and apatite such as hydroxylapatite, fluorapatite, chlorapatite, and/or calcium deficient apatite, and combinations thereof.

Suitable (hydrogel) polymers as base section material include but are not limited to collagen, poly(lactic-co-glycolic acid) (PLGA), polylactic acid (PLA), polycaprolactone (PCL), polyvinyl alcohol (PVA), polyvinyl pyrrolidone (PVP), polyacrylamide, polyurethane, polyethylene glycol (PEG), chitin, poly(hydroxyalkyl methacrylate), water-swellable N-vinyl lactams, starch graft copolymers, and derivatives and combinations thereof.

Other preferred materials for the base section comprise a polyaryletherketone (PAEK) polymer. A PAEK polymer comprises a semi-crystalline thermoplastic polymer containing alternately ketone (R-CO-R) and ether groups (R-O-R). The linking group R between the functional groups comprises a 1,4-substituted aryl group. The PAEK polymer used in the base section may inter alia comprise PEK (polyetherketone), PEEK (polyetheretherketone), PEKK (polyetherketoneketone), PEEKK (polyetheretherketoneketone) and PEKEKK (polyetherketoneetherketoneketone). Due to its excellent resistance to hydrolysis, the polyaryletherketone polymer of the base section is advantageously used in the invented implant. It does not break down when sterilized, nor when implanted in the body for an extended time. It also turns out to bond particularly well to the elastomeric material of the middle and top sections.

The material used in the base section of the invented implant may be used as such, or, in an embodiment, may comprise a reinforcing material selected from the group consisting of fibrous or particulate polymers and/or metals.

The base section of the invented implant may also comprise a contrast agent for medical imaging that absorbs radiation, such as a radiocontrast or MRI contrast agent, or a radiopharmaceutical agent that itself emits radiation. The base section may also comprise a small solid object or body, such as a bead, that may for instance comprise a refractory metal such as tantalum.

The base section of the plug-shaped implant functions as a bone anchor, whereas the combination of middle and top sections functions as partial replacement for the damaged cartilage and as scaffold for cartilage regeneration. In the plug-shaped implant, the top section refers to the section that is closest to the cartilage phase, when implanted. The base section refers to the section that is furthest from the cartilage phase, when implanted. The middle section is situated in between the top and base sections.

The cross-section of the plug-shaped implant through a horizontal or a vertical plane may have any suitable shape. The cross-section may be circular, square or may be polygonal, such as hexagonal, octagonal, or decagonal. In some embodiments, the plug-shaped implant may be tapered such that it is shaped as a truncated cone structure. Preferably, the implant has a smaller cross-section at the base section than at the top section. The cross-section (or diameter in case of a cylindrical implant) may vary continuously between the base and top section, or may show discontinuities, for instance at the interface between sections.

When the implant has a tapered profile, the angle of the taper is preferably between 1° and 45°. In some embodiments, the taper is between about 3° and 30°, more preferably between 5° and 30°, even more preferably between 10° and 15°. A tapered profile may facilitate insertion of the implant into an osteochondral defect and may further reduce possible damage to host tissue. The implant is preferably used without any means of attachment and remains in the osteochondral structure by its geometry and the surrounding tissue structure. The implant may be used in the knee, but may also be used for other joints, such as a temporal-mandibular joint, an ankle, a hip, a shoulder, and the like.

According to the invention, the plug-shaped implant on top of the base section further comprises a middle section configured for replacing cartilage tissue, and a top section configured for growing cartilage tissue onto and into, wherein the middle and top section comprise the same non-biodegradable thermoplastic elastomeric material. By this is meant that at least its building blocks are chemically the same. As mentioned herein below, some physical properties may differ, for instance their weight averaged molecular weight. The non-biodegradable thermoplastic elastomeric material is porous in the top section, and non-porous in the middle section, and comprises a linear block copolymer comprising urethane and urea groups. Moreover, the non-biodegradable thermoplastic elastomeric material is substantially free of an added peptide compound having cartilage regenerative properties. It has surprisingly been found that the implant of the invention is able to regenerate cartilage tissue, thus avoiding the use of any functional compound exhibiting cartilage regenerative properties. In particular, it has been found that the implant according to this embodiment does not need the use of peptides, for instance those comprising an RGD-sequence. These compounds have been said to enable binding integrin's and thereby stimulating cell adhesion.

The linear block copolymers are segmented copolymers with elastic properties that originate from hydrogen bonding interaction between molecular chains. Such copolymers comprise 'hard' crystallized blocks of polyurethane and/or polyurea segments, and may also comprise 'hard' crystallized blocks of polyester and/or polyamide between 'soft' blocks. At room temperature, the low melting 'soft' blocks may be incompatible with the high melting 'hard' blocks, which induces phase separation by crystallization or liquid-liquid demixing. These copolymers exhibit reversible physical crosslinks that originate from crystallization of the 'hard' blocks of the segmented copolymer. The thermoplastic elastomers may be formed into any shape at higher temperatures, more in particular at temperatures above the melting point of the 'hard' blocks. On the other hand, the thermoplastic elastomers provide mechanical stability and elastic properties at low temperatures, i.e. at typical body temperatures. This makes these materials particularly suitable as replacement material for human or animal cartilage.

The constituents of the non-biodegradable thermoplastic elastomer may generally comprise three building blocks: a long-chain diol, for example with a polyether, polyester or polycarbonate backbone, a bifunctional di-isocyanate, and, finally, a chain extender, such as water, another (sometimes short-chain) diol, or a diamine. The latter chain extender is preferred since this leads to bisurea units in the thermoplastic elastomer.

An embodiment of the implant wherein the non-biodegradable thermoplastic elastomeric material is aliphatic is preferred. This means that all building blocks of the thermoplastic elastomer are devoid of aromatic groups and contain aliphatic groups only. The non-biodegradable thermoplastic elastomer may be prepared in a one pot procedure, in which a long-chain diol is first reacted with an excess of a di-isocyanate to form an isocyanate-functionalized prepolymer. The latter is subsequently reacted with a chain extender, such as the preferred diamine, which results in the formation of a higher molecular weight thermoplastic elastomeric polymer containing urethane groups. If a diamine is used as the chain extender, the thermoplastic elastomer will also contain bisurea groups, which is preferred.

The synthetic procedure to prepare the non-biodegradable thermoplastic elastomers may lead to a distribution in the 'hard' block lengths. As a result, the phase separation of these block copolymers may be incomplete, in that part of the 'hard' blocks, in particular the shorter ones, are dissolved in the soft phase, causing an increase in the glass transition temperature. This is less desired for the low temperature flexibility and elasticity of the thermoplastic elastomeric material of the top and middle sections. The polydispersity in `hard' blocks shows as a broad melting range, and a rubbery plateau in dynamic mechanical thermal analysis (DMTA) that is dependent on temperature. Preferred embodiments therefore comprise elastomeric block copolymers containing 'hard' blocks of substantially uniform length. These may be prepared by fractionation of a mixture of 'hard' block oligomers, and subsequent copolymerization of the uniform 'hard' block oligomers of a specific length (or length variation) with the prepolymer, mentioned above.

Although the thermoplastic elastomers may be prepared by a chain extension reaction of an isocyanate-functionalized prepolymer with a diamine, they may also be prepared by a chain extension reaction of an amine-functionalized prepolymer with a di-isocyanate. Examples of suitable, commercially available diamines and di-isocyanates include alkylene diamines and/or di-isocyanates, arylene diamines and/or di-isocyanates. Amine-functionalized prepolymers are also commercially available, or can be prepared from (readily available) hydroxy functionalized prepolymers by cyanoethylation followed by reduction of the cyano-groups, by Gabriel synthesis (halogenation or tosylation followed by modification with phthalimide, and finally formation of the primary amine by deprotection of the phthalimide group) or by other methods that are known in the art. Isocyanate-functionalized prepolymers can be prepared by reaction of hydroxy functionalized prepolymers with di-isocyanates, such as for example isophorone di-isocyanate (IPDI), 1,4-diisocyanato butane, 1,6-diisocyanato hexane or 4,4'-methylene bis(phenyl isocyanate). Alternatively, isocyanate-functionalized prepolymers can be prepared from amine-functionalized prepolymers, for example by reaction with di-tert-butyl tricarbonate. Hydroxy-functionalized prepolymers of molecular weights typically ranging from about 500 g/mol to about 5000 g/mol of all sorts of compositions are also advantageously used. Examples include prepolymers of polyethers, such as polyethylene glycols, polypropylene glycols, poly(ethylene-co-propylene) glycols and poly(tetrahydrofuran), polyesters, such as poly(caprolactone)s or polyadipates, polycarbonates, polyolefins, hydrogenated polyolefins such as poly(ethylene-butylene)s, and the like. Polycarbonates are preferred.

Particularly preferred are prepolymers of polycarbonates. Such prepolymers yield an implant according to an embodiment, wherein the non-biodegradable thermoplastic elastomeric material further comprises carbonate groups, besides the urethane and urea groups. Such an implant has proven to better fulfill the aims of the present invention than other implants. In particular, it has proven to be beneficial in that its mechanical properties are well adapted to the mechanical properties of human or animal cartilage. Surprisingly, regeneration of cartilage is improved when using this embodiment in an implanted implant.

A particularly preferred embodiment of the invention provides an implant, wherein the non-biodegradable thermoplastic elastomeric material comprises a poly-urethane-bisurea-alkylenecarbonate, more preferably a poly-urethane-bisurea-hexylenecarbonate.

Apart from disclaiming a peptide compound having cartilage regenerative properties in the linear block copolymer, the implant may comprise agents that facilitate migration, integration, regeneration, proliferation, and growth of cells into and around the implant or patch composition, and/or the injury or defect, and/or promote healing of the injury or defect, and/or are chondrogenic and osteogenic, i.e., build, grow and produce cartilage and bone, respectively. These agents, include but are not limited to cytokine compounds, chemokine compounds, chemo attractant compounds, anti-microbial compounds, anti-viral compounds, anti-inflammatory compounds, pro-inflammatory compounds, bone or cartilage regenerator molecules, cells, blood components (e.g., whole blood and platelets), and combinations thereof. Agents that increase strength and facilitate attachment can also be included in the implant. In a preferred embodiment, the non-biodegradable elastomeric linear block copolymer does not comprise any compound having cartilage regenerative properties.

With a substantially non-porous material in the context of the present invention is meant a material having a porosity of less than 20 %, relative to the total volume of the material, preferably up to 10%, more preferably up to 5%, and more preferably still up to 1% of the total volume of the material. A porous material comprises pores, which are defined as minute openings. The pores may be micropores, having a diameter of less than 1 mm, and may be macropores, having a diameter of greater than 1 mm. The pores may be interconnected, which is preferred, and which means that pores are internally connected or there is continuity between parts or elements. A non-porous material in the context of the present invention does not mean a material that is impermeable to molecules of any size, and some small molecules may indeed be able to pass through the non-porous material. Rather, a non-porous material in the context of the present invention represents a material that is impermeable to synovial fluid and/or blood.

Pore sizes in the porous parts of the implant may be chosen from 100-1000 micron, more preferably from 100-500 micron, and most preferably from 300-500 micron.

The non-biodegradable thermoplastic elastomer used in the top and middle sections of the implant is particularly advantageous since it allows adapting its mechanical properties to those of human and animal cartilage. In an embodiment of the invention, an implant may be provided wherein the elastomeric material of the middle section has an elastic modulus at room temperature of less than 10 MPa, more preferably of less than 8 MPa, of less than 7 MPa, of less than 6 MPa, of less than 5 MPa, of less than 4 MPa, of less than 3 MPa, or of less than 2 MPa.

In the context of the present application, room temperature is meant to be a temperature in the range of 20-30°C, more preferably 25°C.

Likewise, preferred embodiments of the implant comprise a top section wherein the porous non-biodegradable elastomeric material of the top section has an elastic modulus at room temperature of less than 80% of the elastic modulus of the non-biodegradable elastomeric material of the middle section, more preferably of less than 50%, even more preferably of between 10-50%, even more preferably of between 15-40%, and most preferably of between 20-30% of the elastic modulus of the non-biodegradable elastomeric material of the middle section. Such a reduced elastic modulus may be achieved by modifying the porosity of the material of the middle section, or by modifying physical properties of the material in the middle section through changing its weight average molecular weight for instance.

The porosity of the non-biodegradable elastomeric material of the top section may be chosen within a broad range. Preferred porosities of the non-biodegradable elastomeric material of the top section are selected from 20-80% by volume, more preferably from 30-70% by volume, even more preferably from 40-60% by volume, and most preferably from 45-55% by volume.

A useful embodiment of the invention provides an implant, wherein the base section comprises a core of non-porous base section material and a circumferential shell of porous base section material, wherein the circumferential shell has a thickness that is less than 10% of a largest diameter of the base section. Other useful embodiments provide an implant wherein the circumferential shell has a thickness of less than 9%, of less than 8%, of less than 7%, of less than 6%, of less than 5%, of less than 4%, of less than 3%, of less than 2%, or of less than 1% of a largest diameter of the base section. The cross-sectional area of the circumferential shell covers at most 35% of a largest cross-sectional area of the base section. Other useful embodiments provide an implant wherein the cross-sectional area of the circumferential shell is less than 30%, less than 25%, less than 20%, less than 15%, less than 10%, less than 5%, less than 3%, or less than 1% of a largest cross-sectional area of the base section.

Embodiments having the above-disclosed preferred combinations of mechanical properties of the top and middle section tend to promote regeneration of cartilage. This is believed to be due to a favorable stress (re)distribution of the osteochondral structure including the implant during (dynamic) loading.

Another embodiment of the invention provides an implant, wherein the base section extends between a top surface and a bottom surface, and comprises a layer of porous base section material, wherein the layer is adjacent to the top surface and has a thickness that is less than 10% of a largest height of the base section, and wherein pores of the base section material in the layer comprise the biocompatible elastomeric material, preferably all pores. In other embodiments, the layer that is adjacent to the top surface has a thickness of less than 10%, of less than 8%, of less than 6%, of less than 5%, of less than 4%, of less than 3%, of less than 2%, or of less than 1% of a largest height of the base section. All the above embodiments may improve the adhesion of the middle section (and top section) to the base section to varying degrees. At the same time, the mechanical properties of the base section, and the support offered by the base section to the implant, remain at an adequate level.

Another embodiment of the invention relates to an implant, comprising a substantially non-porous polyaryletherketone polymer with a porosity of less than 20 %, relative to the total volume of the polyaryletherketone polymer.

Yet another embodiment provides an implant wherein the base section comprises a non-porous polyaryletherketone polymer.

In another embodiment of the invention, the top surface of the base section of the implant comprises irregularities or undulations. Irregularities may for instance comprise ridges having a saw-toothed shape. Undulations may be irregular or regular, such as those having a sinusoidal shape.

Another useful embodiment relates to an implant, wherein the base section comprises a centrally located cavity that comprises the biocompatible elastomeric material. Such a cavity may further improve the adhesion of the middle section (and top section) to the base section. The cavity may be cylindrical, or its cross-section may be square, or polygonal. The walls of the cavity may also be provided with irregularities or undulations, or may comprise sections of a larger cross-sectional area than its average cross-sectional area. Several of such cavity sections may be provided at different heights of the base section to form mechanical locking structures.

Yet another embodiment provides an implant, wherein the base section comprises an outer surface having irregularities or undulations. Such outer surface irregularities may for instance comprise ridges having a saw-toothed shape, for instance extending circumferentially over (part of) the outer surface of the base section. Undulations may be irregular or regular, such as those having a sinusoidal shape. The undulations may likewise extend circumferentially over (part of) the outer surface of the base section. Irregularities and undulations may be provided by casting the materials in a suitably profiled mold, or, alternatively, may be provided by mechanical machining, for instance by rotary milling of a molded implant.

A useful embodiment of the invention provides an implant, wherein the middle section comprises a core of non-porous elastomeric material and a circumferential shell of porous elastomeric material, wherein the shell has a thickness that is less than 10% of a largest diameter of the middle section. Other useful embodiments provide an implant wherein the circumferential shell has a thickness of less than 9%, of less than 8%, of less than 7%, of less than 6%, of less than 5%, of less than 4%, of less than 3%, of less than 2%, or of less than 1% of a largest diameter of the middle section. The largest diameter is for instance appropriate in an embodiment wherein the plug-shaped implant is tapered and has circular cross-sections. Alternatively, the cross-sectional area of the circumferential shell covers at most 35% of a largest cross-sectional area of the middle section. Other useful embodiments provide an implant wherein the cross-sectional area of the circumferential shell is less than 30%, less than 25%, less than 20%, less than 15%, less than 10%, less than 5%, less than 3%, or less than 1% of a largest cross-sectional area of the middle section. The largest cross-sectional area is for instance appropriate in an embodiment wherein the plug-shaped implant is tapered.

The height of the plug-shaped implant may be chosen according to the specific application in the body. Heights may vary from 3 to 18 mm for instance. According to a useful embodiment of the invention, an implant is provided wherein a height of the base section, a height of the non-porous middle section, and a height of the porous top section are selected such that a top surface of the implant comes to lie below a top surface of cartilage present on an osteochondral structure when implanted, preferably over a distance of between 0.1 - 1 mm. This embodiment promotes growing cartilage tissue into, but also onto the top section, whereby a strong fixation is built between the top section and the newly formed cartilage. It has turned out that cartilage cells from the host cartilage have a strong affinity for the segmented elastomer of the top section, and therefore are prone to colonize the surface thereof to produce new hyaline cartilage tissue on top of the implant.

Another embodiment provides an implant wherein a height of the base section, a height of the non-porous middle section, and a height of the porous top section are selected such that a bottom surface of the middle section comes to lie about level with a bottom surface of cartilage present on an osteochondral structure when implanted.

Yet another embodiment of the invention provides a top section, a top surface of which is slightly curved. Preferred radii of curvature of the top surface of the top section in a sagittal plane are selected to range from 15 - 150 mm, more preferably from 17 - 125 mm, even more preferably from 19 - 100 mm, even more preferably from 21 - 75 mm, even more preferably from 23 - 50 mm, and most preferably from 25 - 30 mm. This embodiment may regenerate a new cartilage layer on the top surface of the top section of the implant of about equal thickness across the top surface. The result may be a radius of a top surface of the regenerated cartilage that is about the same as the radius of the surrounding native cartilage layer next to the implant, thereby showing a continuity in radius. The top surface of the top section of the implant may also be curved in a medial-lateral plane, preferably with a radius of curvature with the ranges disclosed above for the sagittal plane. In a practical embodiment, the top surface of the top section of the implant has a radius of curvature that is equal in the sagittal and the medial-lateral plane. This embodiment thus comprises a spherical top surface.

Another aspect of the invention provides a method for the preparation of the implant as defined hereinbefore, said method comprising the steps of:
a) providing in a mold at room temperature a base section that comprises base section material comprising one of a biocompatible metal, ceramic, mineral, such as phosphate mineral, and non-biodegradable polymer, optionally a hydrogel polymer, and combinations thereof; and granules of a non-biodegradable thermoplastic elastomeric material on top of the base section, the non-biodegradable thermoplastic elastomeric material comprising a linear block copolymer comprising urethane and urea groups, and substantially free of an added peptide compound having cartilage regenerative properties;
b) closing the mold and heating the above assembly to a temperature of between 100°C and 250°C under a pressure of between 1 and 2 GPa, such that the non-biodegradable thermoplastic elastomeric material melts and fuses with the base section; and
c) cooling the assembly to room temperature to consolidate the non-biodegradable thermoplastic elastomeric material and opening the mold;
d) providing a top section of the non-biodegradable thermoplastic elastomeric material with pores either before or after opening the mold.

In a preferred embodiment, step (a) of the method comprises providing a base section that comprises a substantially non-porous polyaryletherketone polymer with a porosity of less than 20 % relative to the total volume of the polyaryletherketone polymer; and granules of the non-biodegradable thermoplastic elastomeric material on top of the base section in a mold at room temperature.

In another embodiment of the method, the mold is opened after step b) and additional granules of the non-biodegradable thermoplastic elastomeric material are added to the mold, and step b) is repeated. The amount of material added in the two-step embodiment of the method may be chosen within wide ranges. Increasingly good results are obtained when the ratio between the first addition and the second addition of granules of the non-biodegradable thermoplastic elastomeric material is selected from 01:99 to 99:01, more preferably from 30:70 to 97:03, and most preferably from 70:30 to 95:05.

In another embodiment of the method, the heating temperature of step b) is between 110°C and 225°C, more preferably between 120°C and 200°C, and most preferably between 130°C and 175°C. Preferred pressures at all cited temperature ranges are between 1.1 and 1.8 GPa, and more preferably between 1.2 and 1.6 GPa.

A method for the preparation of a thermoplastic elastomeric material comprising a linear block copolymer comprising urethane and urea groups, and being substantially free of an added peptide compound having cartilage regenerative properties is described, comprising:
- preparing an isocyanate-terminated prepolymer by reacting a diol with a di-isocyanate,
- polymerizing the isocyanate-terminated prepolymer by chain extension with a diamine;
wherein the above steps are carried out under the exclusion of a peptide compound having cartilage regenerative properties, more preferably under the exclusion of any compound having cartilage regenerative properties.

In an example, the diol is selected from a polyester diol, a polyether diol and, preferably, a carbonate diol, and combinations thereof.

In another example, the diisocyanate comprises an n-alkylene-diisocyanate.

In yet another example, the diamine comprises a primary diamine, preferably an n-alkylene-diamine.

### BRIEF DESCRIPTION OF THE FIGURES

The invention will now be further elucidated by the following figures and examples. In the figures:
Figures 1A and 1C show a schematic side view of implants in the form of a plug;
Figures 1B and 1D shows a schematic side view of an exemplary implant according to the present invention;
Figure 2A shows a schematic perspective view of a base section;
Figure 2B shows a schematic cross-section of the embodiment of figure 2A;
Figures 2C and 2D show a schematic detailed view of parts B and C of figure 2B;
Figure 3 shows a schematic representation of a possible synthetic route to a thermoplastic polycarbonate material;
Figure 4 shows a ¹H-NMR spectrum of the thermoplastic polycarbonate material prepared in Example 1;
Figures 5A to 5C show DSC thermograms of the thermoplastic polycarbonate material prepared in Example 1 at different heating rates;
Figures 6A to 6C show a schematic representation of a defect in an osteochondral structure (6A), the osteochondral structure comprising an implant (6B) and the same osteochondral structure after on-/ingrowth of cartilage (6C);
Figures 7A and 7C show a schematic side view of implants in the form of a plug;
Figures 7B and 7D shows a schematic side view of an implant according to yet another embodiment of the present invention; and finally
Figures 8A to 8C show a schematic representation of a defect in an osteochondral structure (8A), the osteochondral structure comprising an implant (8B) and the same osteochondral structure after on-/ingrowth of cartilage (8C).

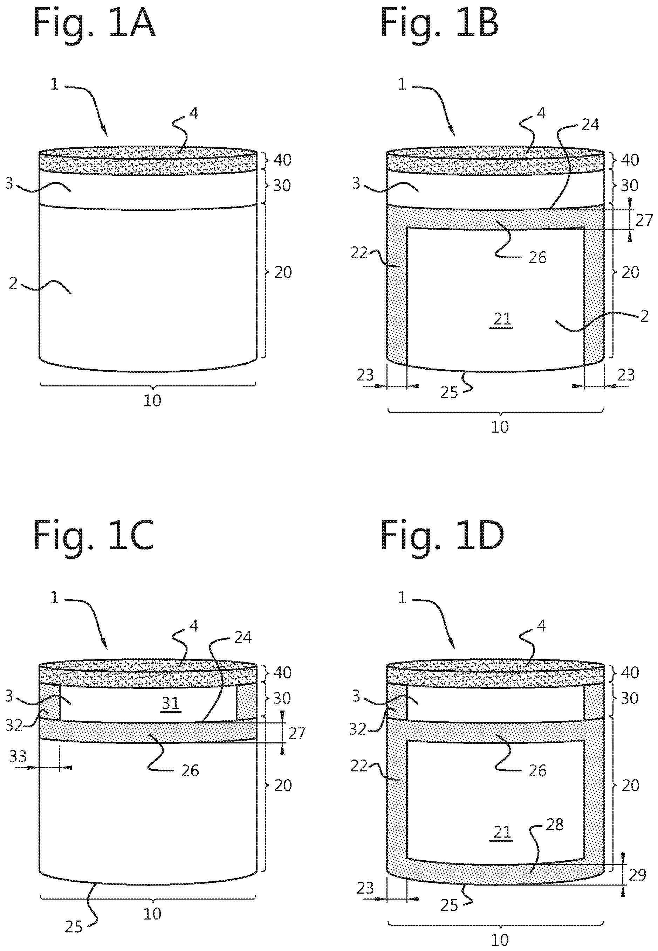

Referring to figure 1A, a side view of an implant is shown. The implant 1 in the shape of a plug comprises a base section 2, configured for anchoring in bone tissue, a middle section 3 configured for replacing cartilage tissue, and a top section 4 configured for growing cartilage tissue onto and into. The middle section 3 and top section 4 comprise the same thermoplastic elastomeric material. The thermoplastic elastomeric material in this embodiment comprises a poly-urethane-bisurea-hexylenecarbonate, the preparation and properties whereof will be elucidated further below. The top section 4 however comprises poly-urethane-bisurea-hexylenecarbonate in porous form, whereas the middle section 3 comprises the same poly-urethane-bisurea-hexylenecarbonate without any pores. The base section 2 comprises a non-porous polyaryletherketone polymer, which, in the implant shown is a non-porous PEKK polymer. The implant 1 is cylindrical and has a diameter 10 of 6 mm. The height 20 of the base section 2, the height 30 of the middle section 3, and the height 40 of the top section 4 add up to a total height of 6 mm.

Figure 1B schematically represents a side view of an embodiment of an implant according to the present invention. The embodied implant 1 in the shape of a plug again comprises a base section 2, configured for anchoring in bone tissue, a middle section 3 configured for replacing cartilage tissue, and a top section 4 configured for growing cartilage tissue onto and into. The middle section 3 and top section 4 comprise the same poly-urethane-bisurea-hexylenecarbonate material, which is porous in the top section 4, and non-porous in the middle section 3. The base section 2 comprises a substantially non-porous PEKK polymer with a porosity of less than 20 %, relative to the total volume of the PEKK polymer. The base section 2 of this embodiment in particular comprises a core 21 of non-porous PEKK polymer and a circumferential shell 22 of porous PEKK polymer. The shell 22 has a thickness 23 of about 8% of the diameter 10 of the base section 2 (and implant 1). The base section 2 further extends between a top surface 24 and a bottom surface 25, and comprises a layer 26 of porous PEKK polymer, which layer 26 is adjacent to the top surface 24 and has a thickness 27 of about 8% of the height 20 of the base section 2. The pores of the PEKK polymer in the layer 26 comprise the biocompatible poly-urethane-bisurea-hexylenecarbonate which originates from the middle section 3 and has infiltrated the pores of the PEKK polymer in the layer 26 during manufacturing. A method for manufacturing the implant will be elucidated further below. As with the embodiment of figure 1A, the implant 1 is cylindrical and has a diameter 10 of 6 mm. The height 20 of the base section 2, the height 30 of the middle section 3, and the height 40 of the top section 4 add up to a total height of 6 mm.

Figure 1C schematically represents a side view of an implant. The embodied implant 1 in the shape of a plug again comprises a base section 2, configured for anchoring in bone tissue, a middle section 3 configured for replacing cartilage tissue, and a top section 4 configured for growing cartilage tissue onto and into. The middle section 3 and top section 4 comprise the same poly-urethane-bisurea-hexylenecarbonate material, which is porous in the top section 4, and substantially non-porous in the middle section 3. The base section 2 comprises a substantially non-porous PEKK polymer with a porosity of less than 20 %, relative to the total volume of the PEKK polymer. The base section 2 of this implant in particular extends between a top surface 24 and a bottom surface 25, and comprises a layer 26 of porous PEKK polymer, which layer 26 is adjacent to the top surface 24 and has a thickness 27 of about 8% of the height 20 of the base section 2. The pores of the PEKK polymer in the layer 26 comprise the biocompatible poly-urethane-bisurea-hexylenecarbonate which originates from the middle section 3 and has infiltrated the pores of the PEKK polymer in the layer 26 during manufacturing. The middle section 3 of this implant in particular comprises a core 31 of non-porous poly-urethane-bisurea-hexylenecarbonate polymer and a circumferential shell 32 of porous poly-urethane-bisurea-hexylenecarbonate polymer. The shell 32 has a thickness 33 of about 8% of the diameter 10 of the middle section 3 (and implant 1). The base section 2 further extends between a top surface 24 and a bottom surface 25, and comprises a layer 26 of porous PEKK polymer, which layer 26 is adjacent to the top surface 24 and has a thickness 27 of about 8% of the height 20 of the base section 2. The dimensions and shape are the same as in the implants of figures 1A and 1B.

Figure 1D schematically represents a side view of yet another embodiment of an implant according to the present invention. The embodied implant 1 in the shape of a plug corresponds to the one shown in figure 1C. In addition, the middle section 3 of this embodiment now has a circumferential shell 32 of porous poly-urethane-bisurea-hexylenecarbonate polymer having a thickness 33 of about 10% of the diameter 10 of the middle section 3 (and implant 1). Further, the base section 2 comprises a layer 26 of porous PEKK polymer, which layer 26 is adjacent to the top surface 24 and has a thickness 27 of about 5% of the height 20 of the base section 2. The pores of the PEKK polymer in the layer 26 comprise the biocompatible poly-urethane-bisurea-hexylenecarbonate which originates from the middle section 3 and has infiltrated the pores of the PEKK polymer in the layer 26 during manufacturing. The base section 2 further comprises a core 21 of non-porous PEKK polymer and a circumferential shell 22 of porous PEKK polymer. The shell 22 has a thickness 23 of about 5% of the diameter 10 of the base section 2 (and implant 1).

Finally, the base section 2 also comprises a layer 28 of porous PEKK polymer, which layer 28 is adjacent to the bottom surface 25 and has a thickness 29 of about 5% of the height 20 of the base section 2. The dimensions and shape are the same as in the implants of figures 1A to 1C.

Please note that in figures 1B, 1C, and 1D the circumferential shells (22, 32) are shown in cross-section to show their respective thicknesses (23, 33). In a side view, they would extend over the complete diameter 10 of the implant 1.

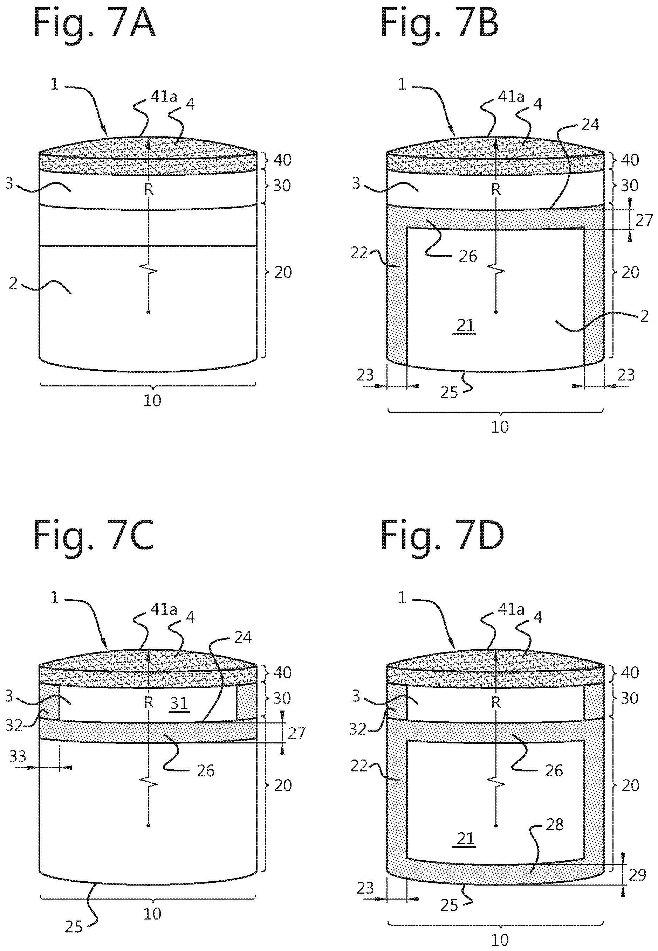

Referring to figure 7A, a side view of another implant is shown. The implant 1 in the shape of a plug comprises the same materials and sections as shown in figure 1A. The dimensions of the implant of figure 7A are the same as those of the implant of figure 1A with one exception. Instead of having a flat top surface 41 of the top section 4 (and the implant 1), as in figure 1A, the top surface 41a of the top section 4 is spherical with a radius of curvature R of about 28 mm (not drawn to scale).

Referring to figure 7B, a side view of another embodiment of the implant according to the present invention is shown. The implant 1 in the shape of a plug comprises the same materials and sections as shown in figure 1B. The dimensions of the implant of figure 7B are the same as those of the implant of figure 1B with one exception. Instead of having a flat top surface 41 of the top section 4, as in figure 1B, the top surface 41a of the top section 4 is spherical with a radius of curvature R of about 28 mm (not drawn to scale).

Referring to figure 7C, a side view of another implant invention is shown. The implant 1 in the shape of a plug comprises the same materials and sections as shown in figure 1C. The dimensions of the implant of figure 7C are the same as those of the implant of figure 1C with one exception. Instead of having a flat top surface 41 of the top section 4, as in figure 1C, the top surface 41a of the top section 4 is spherical with a radius of curvature R of about 28 mm (not drawn to scale).

Referring to figure 7D, a side view of another embodiment of the implant according to the present invention is shown. The implant 1 in the shape of a plug comprises the same materials and sections as shown in figure 1D. The dimensions of the implant of figure 7D are the same as those of the implant of figure 1D with one exception. Instead of having a flat top surface 41 of the top section 4, as in figure 1D, the top surface 41a of the top section 4 is spherical with a radius of curvature R of about 28 mm (not drawn to scale).

Again note that in figures 7B, 7C, and 7D the circumferential shells (22, 32) are shown in cross-section to show their respective thicknesses (23, 33). In a side view, they would extend over the complete diameter 10 of the implant 1 (not drawn to scale).

Referring to figures 2A to 2D, an embodiment of a base section 2 of implant 1 is schematically shown. The base section 2 shown is essentially cylindrical-shaped with a diameter 10, and a height 20. The top surface 24 of the base section has a circumferential flat rim part 240 that gradually extends into a centrally located cavity 241. The cavity 241 is provided with locking parts 242 that have a larger diameter than the diameter of the cavity 241. A shown in detail in figure 2C, the locking parts 242 of the cavity 241 are disk-shaped whereby the outer rim of the disk makes an angle 246 with the longitudinal direction 247 of the base section 2 of between 1° and 20°, more preferably between 5° and 15°. The cavity 241 (and parts 242) during manufacturing of the implant fills with part of the biocompatible elastomeric material to provide an adequate locking of the middle section 3 to the base section 2. As discussed above, the base section 2 comprises a PEKK polymer which may be non-porous or substantially non-porous, the latter embodiment including the examples disclosed above. The base section 2 is further seen to comprise an outer surface having irregularities or undulations. In the present embodiment, these comprise circumferential ridges 243 which, in cross-section, are saw-tooth-shaped, as shown in detail in figure 2D. The angle 244 under which the saw-tooth flanks extend with respect to the transverse direction 245 of the base section 2, is preferably between 70° and 85°, more preferably between 75° and 80°.

### PREPARATION OF THE ELASTOMERIC MATERIAL OF THE TOP AND MIDDLE SECTION

### Example 1: Polycarbonate - aliphatic: Poly(hexylene carbonate urethane)-bis-urea biomaterial MVH313, see table 1 below.

This one-pot two-step produced Biomaterial MVH313 was prepared by functionalization of 1.0 molar equivalent of poly(hexylene carbonate) diol (MW = 2000) with 2.0 molar equivalents of 1,6-diisocyanatohexane (step 1), and subsequent chain extension using 1.0 molar equivalent of 1,6-diaminohexane (step 2).

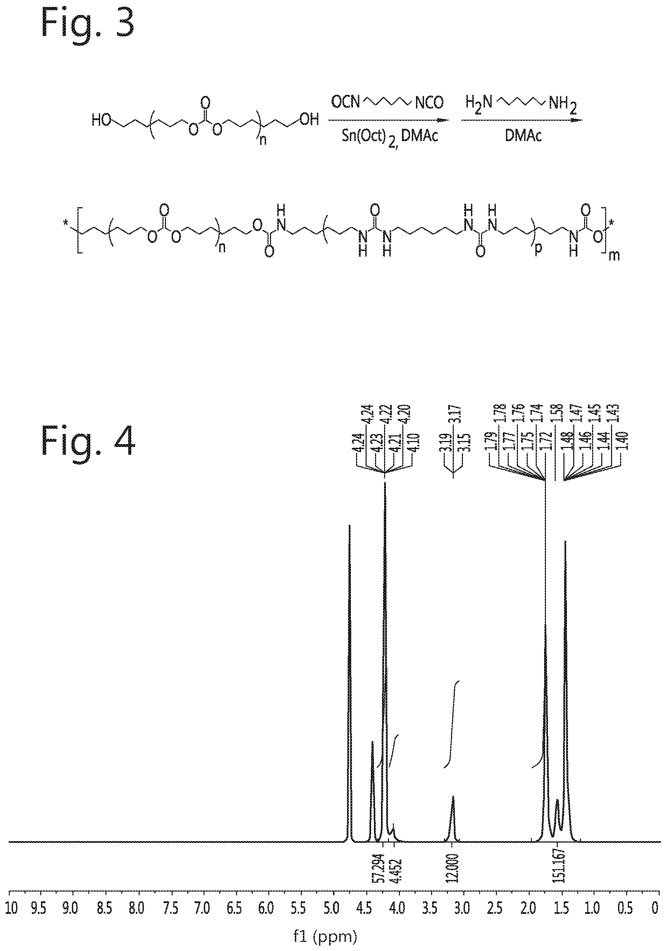

In particular, the aliphatic poly-urethane-urea-hexylene carbonate biomaterial of the middle section 3 and the top section 4 was manufactured as follows (with reference to figure 3). Poly(hexylene carbonate) diol (MW=2000; 23.9 g, 11.9 mmol) was weighed in a 500 mL 3-necked flask and dried by heating to 75 °C overnight under vacuum, after which it was allowed to cool to room temperature. Under an argon atmosphere, 1,6-diisocyanatohexane (4.1 g, 23.9 mmol), DMAc (20 mL) and a drop of Sn(II)bis(2-ethylhexanoate) were added, after which the mixture was heated and stirred for 3 hours upon which the viscosity increased. The mixture was allowed to cool to room temperature, was diluted with DMAc (100 mL) and a solution of 1,6-diaminohexane (1.4 g, 11.9 mmol) in DMAc (50 mL) was added at once under thorough mixing. A gel was immediately formed upon addition and mixing. The mixture was further diluted with DMAc (150 mL) and was heated in an oil bath of 130 °C to acquire a homogeneous viscous slurry. After cooling to room temperature, the mixture was precipitated in a water/brine mixture (2.75 L water + 0.25 L saturated brine) to yield a soft white material. This material was cut into smaller pieces and was stirred in a 1:5 mixture of methanol and water (3 L) for 64 hours. After decanting the supernatant, the resulting solid was stirred in a 2:1 mixture of methanol and water (0.75 L) for 6 hours. Decanting of supernatant, stirring in a 2:1 mixture of methanol and water (0.75 L) for 16 hours, decanting of the supernatant, and drying of the solid at 70 °C in vacuo yielded a flexible, tough elastomeric polymer.

¹H NMR spectroscopy was performed on the resulting polymer, using a Varian 200, a Varian 400 MHz, or a 400 MHz Bruker spectrometer at 298K. DSC was performed using a Q2000 machine (TA Instruments). Heating scan rates of 10 °C/min and 40 °C/min were used for the assessment of the melting temperature (Tm) and the glass transition temperature (Tg), respectively. The Tm was determined by the peak melting temperature and the Tg was determined from the inflection point.

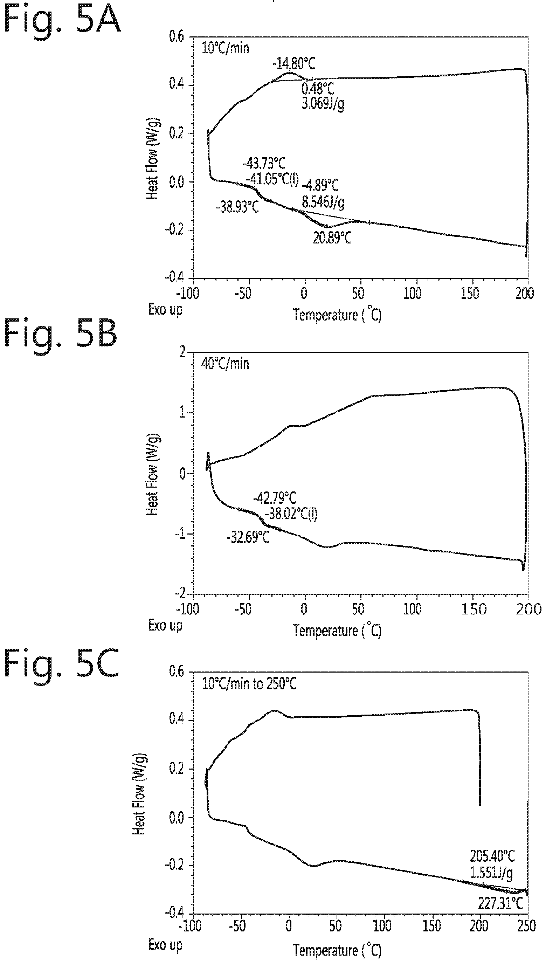

All reagents, chemicals, materials, and solvents were obtained from commercial sources and were used without further purification. The used poly(hexylene carbonate) diol had an average molecular weight of approximately 2 kg/mol. Figures 4 and 5 show the ¹H NMR spectrum and DSC thermograms of the obtained polymer, respectively. The ¹H NMR spectrum results may be summarized as follows: ¹H NMR (400 MHz, HFIP-d2): δ = 4.23 (m, n*4H, n ~ 14.3), 4.10 (m, 4H), 3.17 (m, 12H), 1.87-1.32 (multiple signals for aliphatic CH2 methylenes) ppm. The average molecular weight of the repeating hard/soft block sections is about 2.5 kDa. The DSC results may be summarized as follows: DSC (10 °C/min, figure 5A): Tm (top) = 20.9 °C (soft block melt); DSC (40 °C/min, figure 5B): Tg = -38.0 °C. No second melting point for the hard block was observed up to 200 °C. However, in a final heating run up to 250 °C at 10 °C/min (figure 5C), a small and broad melting transition was observed at ca. 227 °C. In the DSC-diagrams, the endothermic melting peaks are plotted downwards, whereas the exothermic crystallizations are plotted upwards.

The non-porous aliphatic poly-urethane-urea-hexylene carbonate biomaterial had an elastic modulus according to ASTM D638 of 3.6 ± 0.03 MPa.

### Example 2: Polyether - aromatic: Poly(tetrahydrofuran urethane)-bis-urea biomaterial MVH309B, see table 1 below.

In a similar one-pot two-step experimental procedure as described in detail for Biomaterial MVH313, Biomaterial MVH309B was also produced. Particularly, Biomaterial MVH309B was prepared by functionalization of 1.0 molar equivalent of poly-tetrahydrofuran diol (MW = 2000) with 1.33 molar equivalents of bis(4-isocyanatophenyl)methane (MDI) (step 1), and subsequent chain extension using 0.33 molar equivalent of 1,6-diaminohexane (step 2). Biomaterial MVH309B was isolated as a white, flexible, tough elastomeric polymer.

### Example 3: Polyether - aliphatic: Poly(tetrahydrofuran urethane)-bis-urea biomaterial MVH312, see table 1 below.

In a similar one-pot two-step experimental procedure as described in detail for Biomaterial MVH313, Biomaterial MVH312 was also produced. Particularly, Biomaterial MVH312 was prepared by functionalization of 1.0 molar equivalent of poly-tetrahydrofuran diol (MW = 2000) with 2.0 molar equivalents of 1,6-diisocyanatohexane (step 1), and subsequent chain extension using 1.0 molar equivalent of 1,6-diaminohexane (step 2). Biomaterial MVH312 was isolated as a flexible, tough elastomeric polymer.

### Example 4: Polycarbonate - aromatic: Poly(hexylene carbonate urethane)-bis-urea biomaterial MVH311, see table 1 below.

In a similar one-pot two-step experimental procedure as described in detail for Biomaterial MVH313, Biomaterial MVH311 was also produced. Particularly, Biomaterial MVH311 was prepared by functionalization of 1.0 molar equivalent of poly(hexylene carbonate) diol (MW = 2000) with 1.33 molar equivalents of bis(4-isocyanatophenyl)methane (MDI) (step 1), and subsequent chain extension using 0.33 molar equivalent of 1,6-diaminohexane (step 2). Biomaterial MVH311 was isolated as a flexible, tough elastomeric polymer.

### MECHANICAL PROPERTIES OF THE ELASTOMERIC MATERIAL OF THE MIDDLE SECTION

Stress Relaxation Testing was performed on the two aromatic and two aliphatic polymers of Examples 1-4, as well as on three equine cartilage specimens obtained from the Utrecht Medical Centre. A description of the specimens (e.g. polymer classes) and their dimensions are listed in Table 1. Using an Instron Electropulse E10000, each specimen was compressed at a strain rate of 0.005 s-1 up to a strain of 0.05 mm/mm which remained constant for 1800 s. All tests were done in triplicate. During the tests, load, displacement and time were recorded and afterwards, stress relaxation curves were obtained from the data. Stress relaxation is shown by determining the stress relaxation modulus G(t) at the onset of stress relaxation (G(0)) and 1800 s after the onset of stress relaxation (G(1800)) using the following equation: *G*(*t*) = *σ*(*t*) / *ε*₀ , where σ(t) is the compressive stress and ε₀ is the set (constant) strain.

**Table 1: Overview of the stress relaxation tests. All tests were done in triplicate.**

| **Test** | **Code** | **Description** | **Dimensions** |
|---|---|---|---|
| 1 | EC | Equine cartilage | Ø8.5 ×1.55 ± 0.28 mm (average of three specimens) |
| 2 | MVH309B | Polyether based aromatic polymer | 10.8 ×10.5 ×3.0 mm (l × w × h) |
| 3 | MVH311 | Polycarbonate based aromatic polymer | 12.0 × 11.1 × 2.9 mm (l × w × h) |
| 4 | MVH312 | Polyether based aliphatic polymer | 12.4 × 11.3 × 3.0 mm (l × w × h) |
| 5 | MVH313 | Polycarbonate based aliphatic polymer | 13.5 × 13.5 × 3.0 mm (l × w × h) |

The results are shown in Table 2 below.

**Table 2: Stress relaxation moduli of the materials at and after 1800 s after the onset 9 of stress relaxation.**

| **Test** | **Code** | **Stress relaxation modulus [MPa]** | |
|---|---|---|---|
| | | **G₍₀₎** | **G₍₁₈₀₀₎** |
| 1 | EC | 1,32 ± 0,58 | 0.03±0.02 |
| 2 | MVH309B | 0,85±0.04 | 0,65±0,04 |
| 3 | MVH311 | 12,29±0.30 | 10.84±0,39 |
| 4 | MVH312 | 10,36±0,61 | 7,42±0,28 |
| 5 | MVH313 | 3,60±0,03 | 3,14±0,05 |

### PREPARATION OF BIOMATERIAL-CAPPED PEKK BONE ANCHORS

The implant 1 was manufactured by attaching the top and middle sections (4, 3) to a PEKK base section 2 which serves as bone anchor. In a method according to an embodiment of the invention, PEKK bone anchors were capped with the poly-urethane-urea-hexylene carbonate biomaterial by pressing small granules of the aliphatic polycarbonate polymer on top of and into the PEKK anchors. For this purpose, a custom press setup was used. Various temperatures (100 °C to about 150 °C), compressive forces (2 kN to about 4 kN) and methods have been tested. The best results were obtained using a two-step procedure, employing a temperature of 150 °C and using a compressive force of 40 kN (4 tons, or 4000 kg; corresponding to a pressure of 1.4 GPa). Lower temperatures than 150 °C seemed to give less homogenously pressed poly-urethane-urea-hexylene carbonate biomaterial layers (sections 3 and 4), while higher temperatures are less desired as the urea groups in the poly-urethane-urea-hexylene carbonate biomaterial may then degrade to some extent. In the first step, ca. 50 mg of the polymer 12 was pressed onto and into the PEKK bone anchor for 15 minutes, while in the second step, ca. 2 mg of polymer 12 was added to the setup and the sample was pressed for another 15 minutes under the same conditions (150 °C and 40 kN). The samples were subsequently removed from the compression setup and were then allowed to cool. After the second pressing step, the surface of the poly-urethane-urea-hexylene carbonate biomaterial layer (sections 3 and 4) on top of the base section 2 seemed to be substantially flat. The biomaterial was almost transparent and colorless. The edges of the biomaterial showed some fringes or frays, and these were removed using a scalpel.

A central hole (241, 242) of the base section 2 was about 4.5 mm deep and about 2 mm in diameter. The hole was substantially filled with the poly-urethane-urea-hexylene carbonate biomaterial, and the attachment of the biomaterial to the PEKK base section 2 seemed quite strong and robust. Removing the biomaterial from the PEKK base section by force, or loosening the connection at the PEKK-biomaterial interfaces, proved practically impossible. All used equipment and accessories that were intended to come into contact with the PEKK base section 2 and/or with the elastomeric biomaterial were rinsed with ethanol or isopropanol and were thereafter dried. After pressing, and cutting the frays, the PEKK-biomaterial plug implant was rinsed with isopropanol and dried. The plugs may also be produced in a sterilized environment, if needed.

As assessed by measuring, the PEKK base section was 6 mm in diameter and 6 mm tall (a height of 6 mm). The central cavity in the base section was about 2 mm in diameter and about 4.5 mm deep. The elastomeric biomaterial (the aliphatic polycarbonate) positioned onto the PEKK base section was about 6 mm in diameter and about 1 mm high. Accordingly, the total PEKK-biomaterial plug implant was about 7 mm tall.

The top section 4 was provided with pores by drilling holes in it with an average diameter of 300 micron, to a final porosity of 50 vol.%. The porous aliphatic poly-urethane-urea-hexylene carbonate biomaterial of the top section 4 had an elastic modulus according to ASTM D638 of 0.9 ± 0.2 MPa.

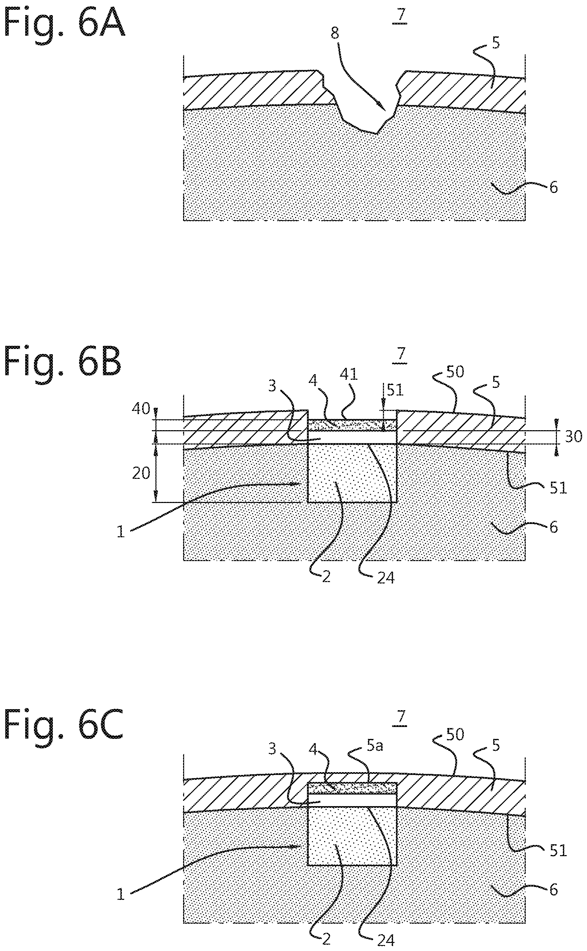

The implant 1 may be implanted into an osteochondral defect 8 as shown in figures 6A to 6C. In a typical method, a cartilage defect extending into the subchondral bone (figure 6 A) is drilled out and a plug-shaped implant 1 is implanted into the drilled hole under some pressure (`press fit'), as shown in figure 6B. Bone then grows onto, and in some embodiments into, the PEKK base section 2, anchoring the implant 1. Surrounding native cartilage 5 grows onto a top side 41 of the top section 4 and new cartilage 5a is generated on top of the implant 1, as shown in figure 6C. As is also shown in figure 6C, the height 20 of the base section 2, the height 30 of the non-porous middle section 3, and the height 40 of the porous top section 4 are selected such that a top surface 41 of the implant 1 comes to lie below a top surface 50 of cartilage 5 present on an osteochondral structure (5, 6) when implanted, preferably over a distance 51 of between 0.1 - 1 mm. In the present case, this distance was about 0.5 mm. The osteochondral structure (5, 6) comprises subchondral bone 6 and a cartilage layer 5 on top of it. A synovial cavity 7 is generally also present.

As also shown in figures 6B and 6C, the height 20 of the base section 2, the height 30 of the non-porous middle section 3, and the height 40 of the porous top section 4 are selected such that a bottom surface 24 of the middle section 3 (or top surface 24 of the base section 2) comes to lie about level with a bottom surface 51 of the cartilage layer 5 of the osteochondral structure (5, 6) when implanted.

### PREPARATION OF BIOMATERIAL-CAPPED METALLIC BONE ANCHORS

Another embodiment of the implant 1 was manufactured by attaching the top and middle sections (4, 3) to a titanium base section 2 which serves as bone anchor. The titanium used was alloy Ti6A14V, which is readily commercially available. The titanium base section was provided with pores having an average pore size of about 300 microns. In a method according to an embodiment of the invention, titanium bone anchors were capped with a poly-urethane-urea-hexylene carbonate biomaterial by pressing small granules of the aliphatic polycarbonate polymer on top of and into the pores of the titanium anchors. For this purpose, the same custom press setup as used in the previous example was used. Optimum results were again obtained using a two-step procedure, employing a temperature of 150 °C and using a compressive force of 40 kN (4 tons, or 4000 kg; corresponding to a pressure of 1.4 GPa). In the first step, ca. 50 mg of the elastomeric polymer was pressed onto and into the titanium bone anchor for 15 minutes, while in the second step, ca. 2 mg of the elastomeric polymer was added to the setup and the sample was pressed for another 15 minutes under the same conditions (150 °C and 40 kN). The samples were subsequently removed from the compression setup and were then allowed to cool. After the second pressing step, the surface of the poly-urethane-urea-hexylene carbonate biomaterial layer (sections 3 and 4) on top of the base section 2 seemed to be substantially flat. The biomaterial was almost transparent and colorless. Some edges of the biomaterial showed fringes or frays, which were removed using a scalpel.

As with the PEKK base anchor, the titanium base anchor was also provided with a central hole (241, 242) with the same dimensions. The hole was substantially filled with the poly-urethane-urea-hexylene carbonate biomaterial, and the attachment of the biomaterial to the titanium base section 2 was satisfactory.

The titanium base section 2 had the same dimensions as the PEKK base section. Since the same mold was used, the elastomeric biomaterial (the aliphatic polycarbonate) positioned onto the titanium base section was about 6 mm in diameter and about 1 mm high. Accordingly, the total titanium-biomaterial plug implant was about 7 mm tall.

The top section 4 was provided with pores by drilling holes in it with an average diameter of 300 micron, to a final porosity of 50 vol.%. The porous aliphatic poly-urethane-urea-hexylene carbonate biomaterial of the top section 4 had an elastic modulus according to ASTM D638 of 0.9 ± 0.2 MPa.

The implant 1 may be implanted into an osteochondral defect 8 as shown in figures 6A to 6C, as was already described above. In a typical method, a cartilage defect extending into the subchondral bone (figure 6 A) is drilled out and a plug-shaped implant 1 is implanted into the drilled hole, as shown in figure 6B. Due to the relatively high stiffness of the titanium base section 2, a press fit was not appropriate. Instead, the dimensions of the drilled out subchondral bone was slightly larger than the dimensions of the titanium base section 2. Bone is seen to grow onto the titanium base section 2, anchoring the implant 1. Surrounding native cartilage 5 grows onto a top side 41 of the top section 4 and new cartilage 5a is generated on top of the implant 1, as shown in figure 6C. As is also shown in figure 6C, the height 20 of the base section 2, the height 30 of the non-porous middle section 3, and the height 40 of the porous top section 4 are selected such that a top surface 41 of the implant 1 comes to lie below a top surface 50 of cartilage 5 present on an osteochondral structure (5, 6) when implanted, preferably over a distance 51 of between 0.1 - 1 mm. In the present case, this distance was about 0.5 mm. The osteochondral structure (5, 6) comprises subchondral bone 6 and a cartilage layer 5 on top of it. A synovial cavity 7 is generally also present.

As also shown in figures 6B and 6C, the height 20 of the base section 2, the height 30 of the non-porous middle section 3, and the height 40 of the porous top section 4 are selected such that a bottom surface 24 of the middle section 3 (or top surface 24 of the base section 2) comes to lie about level with a bottom surface 51 of the cartilage layer 5 of the osteochondral structure (5, 6) when implanted.

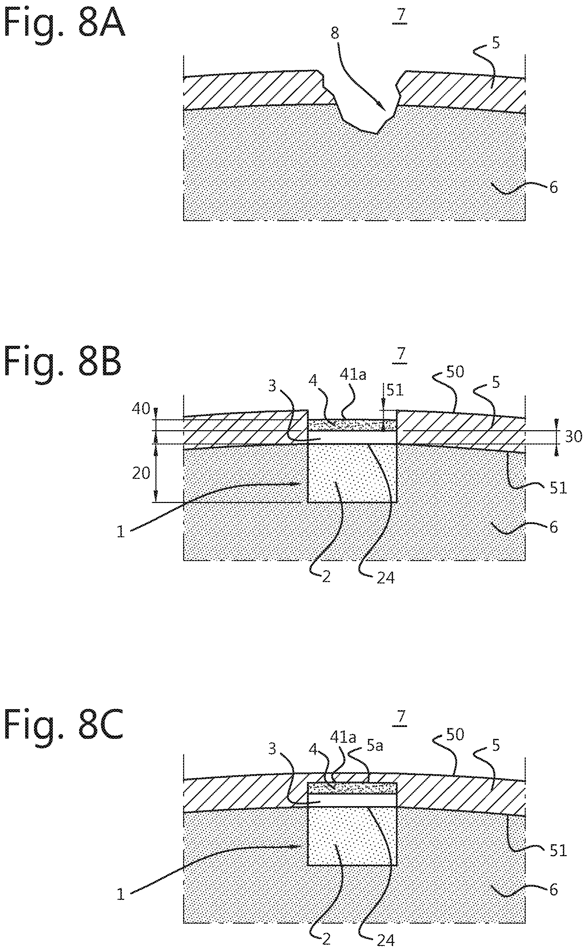

Finally, the implant according to the embodiments shown in figures 7B and 7D may also be implanted into an osteochondral defect 8 as shown in figures 8A to 8C. Due to a spherical top surface 41a of the top layer 4, this embodiment may regenerate a new cartilage layer 5a on the top surface 41a of the top section 4 of the implant 1 of about equal thickness across the top surface 41a. The result may be a radius of a top surface 50 of the regenerated cartilage 5a that is about the same as the radius of the surrounding native cartilage layer 5 next to the implant, thereby showing a continuity in radius.

It will be apparent that many variations and applications are possible for a skilled person in the field within the scope of the invention as defined by the appended claims.

## Claims

1. A non-biodegradable implant (1) for the replacement and regeneration of biological tissue in the shape of a plug, comprising a base section (2) configured for anchoring in bone tissue, a middle section (3) configured for replacing cartilage tissue of an intermediate and deep zone of the cartilage layer and having a thickness of at least 0.2 mm, and a top section (4) configured for growing cartilage tissue onto and into, thus regenerating a superficial zone of the cartilage layer, wherein the middle (3) and top section (4) comprise the same non-biodegradable thermoplastic elastomeric material, which is porous in the top section (4), and non-porous in the middle section (3), wherein the non-biodegradable thermoplastic elastomeric material comprises a linear block copolymer comprising urethane and urea groups, and is substantially free of an added peptide compound having cartilage regenerative properties, and wherein the base section (2) material comprises one of a biocompatible metal, such as titanium or titanium alloy, ceramic, such as sintered crystalline hydroxylapatite, mineral, such as phosphate mineral, and a non-biodegradable polymer, and combinations thereof, wherein the base section (2) comprises a core (21) of non-porous base section material and a circumferential shell (22) of porous base section material, wherein a cross-sectional area of the circumferential shell (22) covers at most 35% of a largest cross-sectional area of the base section (2).

2. Implant according to claim 1, wherein the non-biodegradable thermoplastic elastomeric material further comprises carbonate groups.

3. Implant according to claim 1 or 2, wherein the non-biodegradable thermoplastic elastomeric material comprises a poly-urethane-bisurea-alkylenecarbonate.

4. Implant according to any one of the preceding claims, wherein the porous non-biodegradable thermoplastic elastomeric material of the top section (4) has an elastic modulus at room temperature of less than 80% of the elastic modulus of the non-biodegradable thermoplastic elastomeric material of the middle section (3).

5. Implant according to any one of the preceding claims, wherein the circumferential shell (22) has a thickness that is less than 10% of a largest diameter of the base section (2).

6. Implant according to any one of the preceding claims, wherein the base section (2) extends between a top surface (24) and a bottom surface (25), and comprises a layer (26) of porous base section material, wherein the layer (26) is adjacent to the top surface and has a thickness that is less than 10% of a largest height of the base section (2), and wherein pores of the base section material in the layer (26) comprise the biocompatible elastomeric material.

7. Implant according to any one of the preceding claims, wherein the base section material comprises a metal, selected from titanium, zirconium, chromium, aluminum, stainless steel, hafnium, tantalum or molybdenum, and their alloys, or any combination thereof, and/or a ceramic or mineral, selected from oxides, nitrides, carbides and borides, or any combination thereof.

8. Implant according to any one of the preceding claims, wherein the base section material comprises a non-hydrogel polymer, preferably comprising a polyaryletherketone polymer, such as polyether-ketone-ketone (PEKK), polyether-ether-ketone (PEEK), and polyether-ketone-ether-ketone-ketone (PEKEKK).

9. Implant according to claim 8, comprising a substantially non-porous polyaryletherketone polymer with a porosity of less than 20 %, relative to the total volume of the polyaryletherketone polymer.

10. Implant according to claim 8 or 9, wherein the base section comprises a non-porous polyaryletherketone polymer.

11. Implant according to any one of the preceding claims, further comprising a contrast or radiopharmaceutical agent or body for medical imaging, preferably provided in the base section.

12. Implant according to any one of the preceding claims, wherein the top section (4) has a slightly curved top surface, having a radius of curvature in a sagittal and/or medial-lateral plane ranging from 15 mm to 150 mm.

13. Implant according to any one of the preceding claims, wherein the base section material comprises a reinforcing material selected from the group consisting of fibrous or particulate polymers and/or metals.

14. A method for the preparation of an implant in accordance with any one of the preceding claims, comprising:
a) providing in a mold at room temperature a base section that comprises base section material comprising one of a biocompatible metal, ceramic, mineral, such as phosphate mineral, and non-biodegradable polymer, optionally a hydrogel polymer, and combinations thereof; and granules of a non-biodegradable thermoplastic elastomeric material on top of the base section, the non-biodegradable thermoplastic elastomeric material comprising a linear block copolymer comprising urethane and urea groups, and substantially free of an added peptide compound having cartilage regenerative properties;
b) closing the mold and heating the above assembly to a temperature of between 100°C and 250°C under a pressure of between 1 and 2 GPa, such that the non-biodegradable thermoplastic elastomeric material melts and fuses with the base section; and
c) cooling the assembly to room temperature to consolidate the non-biodegradable thermoplastic elastomeric material and opening the mold;
d) providing a top section of the non-biodegradable thermoplastic elastomeric material with pores either before or after opening the mold.

15. A method according to claim 14, wherein after step b) the mold is opened and additional granules of the non-biodegradable thermoplastic elastomeric material are added to the mold, and step b) is repeated.

## Patentansprüche

1. Ein nicht biologisch abbaubares Implantat (1) für den Ersatz und die Regeneration von biologischem Gewebe in Form eines Stopfens, aufweisend einen für die Verankerung in Knochengewebe ausgebildeten Basisabschnitt (2), einen für das Ersetzen von Knorpelgewebe einer mittleren und tiefen Zone der Knorpelschicht ausgebildeten mittleren Abschnitt (3) mit einer Dicke von mindestens 0,2 mm, und einen oberen Abschnitt (4), ausgebildet, um auf und in einer Oberflächenzone der Knorpelschicht Knorpelgewebe wachsen zu lassen, wodurch eine Oberflächenzone der Knorpelschicht regeneriert wird, wobei der mittlere (3) und der obere Abschnitt (4) dasselbe nicht biologisch abbaubare thermoplastische, im oberen Abschnitt (4) poröse und im mittleren Abschnitt (3) nicht poröse Elastomermaterial aufweisen, wobei das biologisch nicht abbaubare thermoplastische Elastomermaterial ein lineares Blockcopolymer umfasst, welches Urethan- und Harnstoffgruppen aufweist und im Wesentlichen frei ist von einer zugesetzten, knorpelregenerierende Eigenschaften aufweisenden Peptidverbindung, und wobei das Material des Basisabschnitts (2) ein biokompatibles Metall, wie Titan oder eine Titanlegierung, Keramik, wie gesintertes kristallines Hydroxylapatit, ein Mineral, wie ein Phosphatmineral, und ein biologisch nicht abbaubares Polymer oder Kombinationen davon umfasst, wobei der Basisabschnitt (2) einen Kern (21) aus nicht-porösem Basisabschnittsmaterial und eine Ummantelung (22) aus porösem Basisabschnittsmaterial umfasst, wobei eine Querschnittsfläche der Ummantelung (22) höchstens 35% einer größten Querschnittsfläche des Basisabschnitts (2) abdeckt.

2. Implantat nach Anspruch 1, wobei das biologisch nicht abbaubare thermoplastische Elastomermaterial außerdem Carbonatgruppen aufweist.

3. Implantat nach Anspruch 1 oder 2, wobei das biologisch nicht abbaubare thermoplastische Elastomermaterial ein Polyurethan-Bisurea-Alkylencarbonat umfasst.

4. Implantat nach einem der vorherigen Ansprüche, wobei das poröse, nicht biologisch abbaubare thermoplastische Elastomermaterial des oberen Abschnitts (4) bei Raumtemperatur ein Elastizitätsmodul von weniger als 80% des Elastizitätsmoduls des nicht biologisch abbaubaren thermoplastischen Elastomermaterials des mittleren Abschnitts (3) aufweist.

5. Implantat nach einem der vorherigen Ansprüche, wobei die Ummantelung (22) eine Dicke aufweist, die weniger als 10 % des größten Durchmessers des Basisabschnitts (2) beträgt.

6. Implantat nach einem der vorherigen Ansprüche, wobei sich der Basisabschnitt (2) zwischen einer oberen Fläche (24) und einer unteren Fläche (25) erstreckt und eine aus porösem Basisabschnittsmaterial bestehende, an die obere Fläche angrenzende Schicht (26) aufweist, mit einer Dicke, die weniger als 10 % der größten Höhe des Basisabschnitts (2) beträgt, und wobei die Poren des Basisabschnittsmaterials in der Schicht (26) das biokompatible Elastomermaterial aufweisen.

7. Implantat nach einem der vorherigen Ansprüche, wobei das Material des Basisabschnitts ein Metall aufweist, ausgewählt aus der Gruppe, bestehend aus Titan, Zirkonium, Chrom, Aluminium, rostfreiem Stahl, Hafnium, Tantal oder Molybdän und deren Legierungen oder einer Kombination davon, und/oder eine Keramik oder ein Mineral, ausgewählt aus der Gruppe, bestehend aus Oxiden, Nitriden, Carbiden und Boriden oder einer Kombination davon.

8. Implantat nach einem der vorherigen Ansprüche, wobei das Material des Basisabschnitts ein Nicht-Hydrogel-Polymer aufweist, vorzugsweise ein Polyaryletherketon-Polymer, wie Polyetherketonketon (PEKK), Polyetheretherketon (PEEK) und Polyetherketonetherketonketon (PEKEKK).

9. Implantat nach Anspruch 8, aufweisend ein im Wesentlichen nicht poröses Polyaryletherketonpolymer mit einer Porosität von weniger als 20 %, bezogen auf das Gesamtvolumen des Polyaryletherketonpolymers.

10. Implantat nach Anspruch 8 oder 9, wobei der Basisabschnitt ein nicht poröses Polyaryletherketon-Polymer aufweist.

11. Implantat nach einem der vorherigen Ansprüche, ferner aufweisend ein Kontrastmittel oder ein radiopharmazeutisches Mittel oder einen Körper für die medizinische Bildgebung, das bzw. der vorzugsweise im Basisteil vorgesehen ist.

12. Implantat nach einem der vorherigen Ansprüche, wobei der obere Abschnitt (4) in einer sagittalen und/oder medial-lateralen Ebene eine leicht gekrümmte obere Fläche aufweist, mit einem Krümmungsradius im Bereich von 15 mm bis 150 mm.

13. Implantat nach einem der vorherigen Ansprüche, wobei das Material des Basisabschnitts ein Verstärkungsmaterial aufweist, ausgewählt aus der Gruppe, bestehend aus faser- oder teilchenförmigen Polymeren und/oder Metallen.

14. Ein Verfahren zur Herstellung eines Implantats nach einem der vorherigen Ansprüche, aufweisend die folgenden Schritte:
a) Bereitstellen, in einer Form bei Raumtemperatur, eines Basisabschnitts, aus einem Basisabschnittsmaterial, ausgewählt aus der Gruppe, bestehend aus einem biokompatiblen Metall, einer Keramik, einem Mineral, wie z.B. einem Phosphatmineral, und einem nicht biologisch abbaubaren Polymer, gegebenenfalls einem Hydrogelpolymer, und Kombinationen davon, wobei die obere Fläche des Basisabschnitts Körnchen aufweist, aus einem nicht biologisch abbaubaren thermoplastischen Elastomermaterial, wobei das nicht biologisch abbaubare thermoplastische Elastomermaterial ein Urethan- und Harnstoffgruppen aufweisendes lineares Blockcopolymer umfasst, welches im Wesentlichen frei ist von einer knorpelregenerative Eigenschaften aufweisenden zugesetzten Peptidverbindung;
b) Schließen der Form und Erhitzen der obigen Anordnung auf eine Temperatur zwischen 100°C und 250°C bei einem Druck zwischen 1 und 2 GPa, so dass das biologisch nicht abbaubare thermoplastische Elastomermaterial mit dem Basisabschnitt verschmilzt; und
c) Abkühlen der Anordnung auf Raumtemperatur, um das biologisch nicht abbaubare thermoplastische Elastomermaterial zu verfestigen, und Öffnen der Form;
d) Versehen eines oberen Abschnitts des biologisch nicht abbaubaren thermoplastischen Elastomermaterials mit Poren entweder vor oder nach dem Öffnen der Form.

15. Ein Verfahren nach Anspruch 14, wobei nach Schritt b) die Form geöffnet und zusätzliches Granulat des biologisch nicht abbaubaren thermoplastischen Elastomermaterials in die Form gegeben wird und Schritt b) wiederholt wird.

## Revendications

1. Implant non biodégradable (1) en forme de bouchon pour le remplacement et la régénération de tissu biologique, comprenant une section de base (2) configurée pour l'ancrage dans un tissu osseux, une section intermédiaire (3) configurée le remplacement du tissu cartilagineux d'une zone intermédiaire et profonde de la couche cartilagineuse et présentant une épaisseur d'au moins 0,2 mm, et une section supérieure (4) configurée pour y faire croître un tissu cartilagineux en surface et en profondeur, permettant ainsi la régénération d'une zone superficielle de la couche de cartilage, dans lequel la section intermédiaire (3) et la section supérieure (4) comprennent le même matériau élastomère thermoplastique non biodégradable, qui est poreux dans la section supérieure (4), et non poreux dans la section intermédiaire (3), dans lequel le matériau élastomère thermoplastique non biodégradable comprend un copolymère séquencé linéaire comprenant des groupes uréthane et urée, et est sensiblement dépourvu d'un composé peptidique ajouté doté de propriétés de régénération du cartilage, et dans lequel le matériau de section de base (2) comprend l'un d'un métal biocompatible, tel qu'un titane ou un alliage de titane, d'une céramique, telle qu'une hydroxylapatite cristalline frittée, d'un minéral, tel qu'un minéral de phosphate, et d'un polymère non biodégradable, et des combinaisons de ceux-ci, dans lequel la section de base (2) comprend un noyau (21) de matériau non poreux de section de base et une enveloppe circonférentielle (22) de matériau poreux de section de base, dans lequel une superficie en coupe de l'enveloppe circonférentielle (22) couvre au plus 35 % d'une superficie en coupe la plus grande de la section de base (2).

2. Implant selon la revendication 1, dans lequel le matériau élastomère thermoplastique non biodégradable comprend en outre des groupes carbonate.

3. Implant selon la revendication 1 ou 2, dans lequel le matériau élastomère thermoplastique non biodégradable comprend un poly-uréthane-bisurée-alkylènecarbonate.

4. Implant selon l'une quelconque des revendications précédentes, dans lequel le matériau poreux élastomère thermoplastique non biodégradable de la section supérieure (4) a un module d'élasticité à température ambiante inférieur à 80 % du module d'élasticité du matériau élastomère thermoplastique non biodégradable de la section intermédiaire (3).

5. Implant selon l'une quelconque des revendications précédentes, dans lequel l'enveloppe circonférentielle (22) a une épaisseur qui est inférieure à 10 % d'un diamètre le plus grand de la section de base (2).

6. Implant selon l'une quelconque des revendications précédentes, dans lequel la section de base (2) s'étend entre une surface supérieure (24) et une surface inférieure (25), et comprend une couche (26) de matériau poreux de section de base, dans lequel la couche (26) est adjacente à la surface supérieure et a une épaisseur qui est inférieure à 10 % d'une hauteur la plus grande de la section de base (2), et dans lequel les pores du matériau de la section de base dans la couche (26) comprennent le matériau élastomère biocompatible.

7. Implant selon l'une quelconque des revendications précédentes, dans lequel le matériau de section de base comprend un métal, sélectionné parmi le titane, le zirconium, le chrome, l'aluminium, l'acier inoxydable, l'hafnium, le tantale ou le molybdène, et leurs alliages, ou toute combinaison de ceux-ci, et/ou une céramique ou un minéral, sectionné parmi des oxydes, des nitrures, des carbures et des borures, ou toute combinaison de ceux-ci.

8. Implant selon l'une quelconque des revendications précédentes, dans lequel le matériau de section de base comprend un polymère non-hydrogel, de préférence comprenant un polymère de polyaryléthercétone, par exemple de polyéther-cétone-cétone (PEKK), de polyéther-éther-cétone (PEEK), et de polyéther-cétone-éther-cétone-cétone (PEKEKK).

9. Implant selon la revendication 8, comprenant un polymère de polyaryléthercétone sensiblement non poreux avec une porosité inférieure à 20 %, par rapport au volume total du polymère de polyaryléthercétone.

10. Implant selon la revendication 8 ou 9, dans lequel la section de base comprend un polymère de polyaryléthercétone non poreux.

11. Implant selon l'une quelconque des revendications précédentes, comprenant en outre un agent ou corps radiopharmaceutique ou de contraste pour l'imagerie médicale, de préférence fourni dans la section de base.

12. Implant selon l'une quelconque des revendications précédentes, dans lequel la section supérieure (4) a une surface supérieure légèrement incurvée, ayant un rayon de courbure dans un plan sagittal et/ou médio-latéral dans la plage de 15 mm à 150 mm.

13. Implant selon l'une quelconque des revendications précédentes, dans lequel le matériau de section de base comprend un matériau de renforcement sélectionné dans le groupe constitué par des polymères fibreux ou particulaires et/ou des métaux.

14. Procédé de préparation d'un implant selon l'une quelconque des revendications précédentes, comprenant :
a) la fourniture dans un moule à température ambiante d'une section de base qui comprend un matériau de section de base comprenant l'un d'un métal biocompatible, d'une céramique, d'un minéral, tel qu'un minéral de phosphate, et d'un polymère non biodégradable, facultativement un polymère d'hydrogel, et des combinaisons de ceux-ci ; et des granules d'un matériau élastomère thermoplastique non biodégradable sur le dessus de la section de base, le matériau élastomère thermoplastique non biodégradable comprenant un copolymère séquencé linéaire comprenant des groupes uréthane et urée, et sensiblement dépourvu d'un composé peptide ajouté ayant des propriétés de régénération du cartilage ;
b) la fermeture du moule et le chauffage de l'ensemble ci-dessus à une température entre 100 °C et 250 °C sous une pression entre 1 et 2 GPa, de telle sorte que le matériau élastomère thermoplastique non biodégradable fonde et fusionne avec la section de base ; et
c) le refroidissement de l'ensemble à température ambiante pour consolider le matériau élastomère thermoplastique non biodégradable et l'ouverture du moule ;
d) le fait de doter de pores une section supérieure du matériau élastomère thermoplastique non biodégradable avant ou après l'ouverture du moule.

15. Procédé selon la revendication 14, dans lequel après l'étape b) le moule est ouvert et des granules supplémentaires du matériau élastomère thermoplastique non biodégradable sont ajoutés au moule, et l'étape b) est répétée.
